# EUROPEAN PATENT APPLICATION

(11) **EP 3 508 566 A1**
(43) Date of publication of application: **10.07.2019**
(21) Application number: 17846154.7
(22) Date of filing: 17.08.2017
(51) Int. Cl.: C12N 1/12, C12P 7/26

(54) **METHOD FOR CULTURING PHOTOSYNTHETIC MICROALGAE**

(30) Priority: 01.09.2016 JP 2016170925
(71) Applicant: Showa Denko K.K., Tokyo 105-8518 (JP)
(72) Inventor: MATSUMOTO Yoko, Tokyo 105-8518 (JP); ISHIKURA Masaharu, Tokyo 105-8518 (JP); SUZUKI Hiroshi, Tokyo 105-8518 (JP)
(74) Representative: Strehl Schübel-Hopf & Partner
(86) International application number: PCT/JP2017/029513
(87) International publication number: WO 2018/043146

(57) **Abstract**

The present invention provides a method for culturing photosynthetic microalgae with which xanthophyll can be obtained more efficiently than before. The method for culturing photosynthetic microalgae of the present invention comprises: step (A) of increasing the number of cells in which light irradiation (a) of encysted photosynthetic microalgae containing xanthophyll is performed; and step (B) of increasing the xanthophyll content in photosynthetic microalgae in which light irradiation (b) of the photosynthetic microalgae subjected to the step (A) of increasing the number of cells is performed, wherein the light irradiation (a) is light irradiation using as a light source an LED including blue light having a wavelength of 400 to 490 nm, the light irradiation (b) is light irradiation using as a light source an LED including blue light having a wavelength of 400 to 490 nm and an LED including red light having a wavelength of 620 to 690 nm, and the light irradiation (a) and the light irradiation (b) are light irradiations using different light sources.

## Description

### Technical Field

The present invention relates to a method for culturing photosynthetic microalgae containing xanthophyll.

### Background Art

Currently, xanthophyll is used for various purposes.

Astaxanthin that is a type of xanthophyll is a type of red carotenoid, and is known to have a strong antioxidative effect. Thus, astaxanthin is used for pigments for foodstuffs, cosmetic products, health food products and the like.

Astaxanthin can be chemically synthesized, but naturally-derived astaxanthin is widely used. Naturally derived astaxanthin is extracted from shrimps such as krill and northern shrimps, Phaffia rhodozyma, algae and the like.

It is known that the astaxanthin content of the shrimps or Phaffia rhodozyma is low. Thus, a method for obtaining astaxanthin by culturing algae has been studied. It is known that algae such as *Haematococcus* are encysted according to a change in external environment (stress) such as nitrogen source exhaustion or strong light, so that astaxanthin is accumulated in the alga body. Production of astaxanthin from *Haematococcus,* which is currently commercialized, involves a method in which the number of cells is increased by zoospore-like cells having green flagella (green stage) before accumulation of astaxanthin, and astaxanthin is then accumulated in cyst cells (red stage) by stress. However, it is known that in culture of the swarm cells, it is difficult to maintain a culture environment because the swarm cells favor a weak light condition, etc. (see, for example, Non-Patent Literature 1).

In addition, various studies have been conducted on methods for obtaining astaxanthin by culturing algae such as *Haematococcus* (see, for example, Patent Literatures 1 to 4).

For example, Patent Literature 1 discloses a method for producing xanthophyll from photosynthetic microalgae, the method comprising the steps of: inoculating photosynthetic microalgae containing xanthophyll in a nutrient medium; and growing the photosynthetic microalgae; and encysting the grown microalgae.

Patent literature 2 discloses a method for producing green algae, the method comprising performing light irradiation of encysted green algae with a photosynthetic photon flux input of 25,000 µmol/(m³·s) or more.

Patent Literature 3 discloses a method for culturing algae, the method comprising repeatedly irradiating algae with red illumination light and blue illumination light separately and independently.

Patent Literature 4 discloses that in production of astaxanthin in the alga body by culturing algae, light irradiation is performed using a blue LED and a red LED in combination in an astaxanthin production and culture period.

### Citation List

### Patent Literature

Patent Literature 1: International Publication No. WO 2005/116238
Patent Literature 2: Japanese Unexamined Patent Publication No. 2007-97584
Patent Literature 3: International Publication No. WO 2013/021675
Patent Literature 4: International Publication No. WO 2015/151577

### Non Patent Literature

Non Patent Literature 1: Akitoshi Kitamura and two others, "Commercial Production of Astaxanthin from Green Algae of the genus Haematococcus", Bioengineering, Public Interest Incorporated Association, The Society for Biotechnology, Japan, 2015, Vol. 93, No. 7, p. 383-387

### Summary of Invention

### Technical Problem

In Patent Literatures 1 to 4, various studies are conducted for efficiently obtaining xanthophyll such as astaxanthin, but a method for culturing photosynthetic microalgae, with which it is possible to more efficiently obtain xanthophyll, has been desired.

Thus, an object of the present invention is to provide a method for culturing photosynthetic microalgae, with which it is possible to obtain xanthophyll more efficiently than before.

### Solution to Problem

The present inventors have extensively conducted studies, and resultantly found that the above-described object can be achieved by performing light irradiation of encysted photosynthetic microalgae in a specific pattern.

Specifically, the present invention relates to the following items [1] to [10].
[1] A method for culturing photosynthetic microalgae, the method comprising: step (A) of increasing the number of cells in which light irradiation (a) of encysted photosynthetic microalgae containing xanthophyll is performed; and step (B) of increasing the xanthophyll content in photosynthetic microalgae in which light irradiation (b) of the photosynthetic microalgae subjected to the step (A) of increasing the number of cells is performed, wherein
   the light irradiation (a) is light irradiation using as a light source an LED including blue light having a wavelength of 400 to 490 nm,
   the light irradiation (b) is light irradiation using as a light source an LED including blue light having a wavelength of 400 to 490 nm and an LED including red light having a wavelength of 620 to 690 nm, and
   the light irradiation (a) and the light irradiation (b) are light irradiations using different light sources.
[2] The method for culturing photosynthetic microalgae according to [1], wherein where ΔB is a difference between the light amount of blue light in the light irradiation (b) and the light amount of blue light in the light irradiation (a), and ΔR is a difference between the light amount of red light in the light irradiation (b) and the light amount of red light in the light irradiation (a), the relationship of ΔR - ΔB > 0 is satisfied.
[3] The method for culturing photosynthetic microalgae according to [1] or [2], wherein the light irradiation (a) is at least one light irradiation selected from light irradiation (I) using a white LED as a light source, light irradiation (II) using a white LED and a blue LED as a light source, light irradiation (III) using a white LED and a red LED as a light source, light irradiation (IV) using a blue LED and a red LED as a light source, light irradiation (V) using a blue LED and a red LED alternately as a light source, and light irradiation (VI) using a blue LED as a light source, and
   the light irradiation (b) is at least one light irradiation selected from light irradiation (I) using a white LED as a light source, light irradiation (II) using a white LED and a blue LED as a light source, light irradiation (III) using a white LED and a red LED as a light source, light irradiation (IV) using a blue LED and a red LED as a light source, and light irradiation (V) using a blue LED and a red LED alternately as a light source.
[4] The method for culturing photosynthetic microalgae according to any one of [1] to [3], wherein the encysted photosynthetic microalgae containing xanthophyll used in the step (A), are encysted photosynthetic microalgae containing xanthophyll in an amount of 3 to 9% by mass in terms of a dry mass.
[5] The method for culturing photosynthetic microalgae according to any one of [1] to [4], wherein the xanthophyll content of the photosynthetic microalgae is kept at 2% by mass or more in terms of a dry mass in the step (A) and the step (B).
[6] The method for culturing photosynthetic microalgae according to any one of [1] to [5], wherein the photosynthetic photon flux density is 750 µmol/(m²·s) or more in the light irradiation (a) and the light irradiation (b).
[7] The method for culturing photosynthetic microalgae according to any one of [1] to [6], wherein the step (A) is carried out for 3 to 7 days, and the step (B) is carried out for 4 to 10 days, the step (A) and the step (B) are carried out for 7 to 17 days in total.
[8] The method for culturing photosynthetic microalgae according to any one of [1] to [7], wherein the xanthophyll productivity (mg/(L·day)) obtained by dividing the amount of xanthophyll (mg) per 1 L of a culture liquid of photosynthetic microalgae obtained through the step (B) by the total period (days) during which the steps (A) and (B) are carried out is 20 mg/(L·day) or more.
[9] The method for culturing photosynthetic microalgae according to any one of [1] to [8], wherein the xanthophyll is astaxanthin, and the photosynthetic microalga is a green alga of the genus *Haematococcus.*
[10] A culture liquid of photosynthetic microalgae in which the content of xanthophyll obtained by the culture method according to any one of [1] to [9] is 300 mg/L or more.

### Advantageous Effect of Invention

A method for culturing photosynthetic microalgae is provided, with which xanthophyll can be obtained more efficiently than before.

### Brief Description of Drawings

Figure 1 shows a time-dependent change of the total nitrogen concentration in a culture liquid in Example 1 and Comparative Example 1.
Figure 2 shows a time-dependent change of the number of cells in the culture liquid in Example 1 and Comparative Example 1.
Figure 3 shows a time-dependent change of the astaxanthin concentration in the culture liquid in Example 1 and Comparative Example 1.
Figure 4 shows a time-dependent change of the astaxanthin concentration in cells in Example 1 and Comparative Example 1.

### Description of Embodiment

The present invention will now be described specifically.

A method for culturing photosynthetic microalgae according to the present invention comprises step (A) of increasing the number of cells in which light irradiation (a) of encysted photosynthetic microalgae containing xanthophyll is performed; and step (B) of increasing the xanthophyll content in photosynthetic microalgae in which light irradiation (b) of the photosynthetic microalgae subjected to the step (A) of increasing the number of cells is performed. In the present invention, the light irradiation (a) is light irradiation using as a light source an LED including blue light having a wavelength of 400 to 490 nm, the light irradiation (b) is light irradiation using as a light source an LED including blue light having a wavelength of 400 to 490 nm and an LED including red light having a wavelength of 620 to 690 nm, and the light irradiation (a) and the light irradiation (b) are light irradiations using different light sources. Hereinafter, the present invention will be described in detail.

### (Encysted photosynthetic microalgae containing xanthophyll)

In the present invention, encysted photosynthetic microalgae containing xanthophyll are used. The photosynthetic microalgae for use in the present invention should be those that contain xanthophyll, and are encysted.

### (Photosynthetic microalgae)

The photosynthetic microalgae are not particularly limited as long as they are algae which have an ability to produce xanthophyll, can be encysted, and are capable of performing photosynthesis. The photosynthetic microalgae are preferably green algae from the viewpoint of xanthophyll productivity.

As green algae, for example, green algae belonging to the genus *Haematococcus* are preferably used. Examples of the algae of the genus *Haematococcus* include *Haematococcus pluvialis* (*H. pluvialis*), *Haematococcus lacustris* (*H. lacustris*), *Haematococcus capensis* (*H. capensis*), *Haematococcus droebakensi* (*H. droebakensi*) and *Haematococcus zimbabwiensis* (*H. zimbabwiensis*).

Examples of the *Haematococcus pluvialis* (*H. pluvialis*) include UTEX 2505 strain deposited in the Culture Collection of Algae at the University of Texas in the US, and K0084 strain stored in Scandinavian Culture Center for Algae and Protozoa, Botanical Institute at University of Copenhagen in Denmark.

Examples of the *Haematococcus lacustris* (*H. lacustris*) include NIES 144 strain, NIES 2263 strain, NIES 2264 strain and NIES 2265 strain deposited in Public Interest Incorporated Association, National Institute for Environmental Studies, and ATCC 30402 strain and ATCC 30453 strain deposited in ATCC or UTEX 16 strain and UTEX 294 strain.

Examples of the *Haematococcus capensis* (*H. capensis*) include UTEX LB 1023 strain.

Examples of the *Haematococcus droebakensi* (*H. droebakensi*) include UTEX LB 55 strain.

Examples of the *Haematococcus zimbabwiensis* (*H. zimbabwiensis*) UTEX LB 1758 strain.

Among them, *Haematococcus lacustris* and *Haematococcus pluvialis* are preferably used as the photosynthetic algae.

### (Xanthophyll)

The xanthophyll is a type of carotenoid. Examples of the xanthophyll include astaxanthin, canthaxanthin, zeaxanthin, adonirubin, adonixanthin and cryptoxanthin.

In the culture method of the present invention, owing to step(A), the number of cells of photosynthetic microalgae is increased even under irradiation with strong light because it is not necessary to grow cells using floating cells difficult to culture (photosynthetic microalgae which have not been encysted). Since cells grown in step (A) already contain xanthophyll, the content of xanthophyll in each cell of each photosynthetic microalga is increased without damaging the cells owing to step(B), and therefore the culture method of the present invention makes it possible to obtain a large amount of xanthophyll.

The resulting xanthophyll depends mainly on the type of photosynthetic microalgae, and is not particularly limited, but the xanthophyll is preferably astaxanthin which has a high antioxidative effect from the viewpoint of effective utilization of xanthophyll. Examples of the photosynthetic microalgae from which astaxanthin can be obtained include the above-described green algae of the genus *Haematococcus,* and *Haematococcus lacustris* and *Haematococcus pluvialis* are preferable.

### (Encystment)

Stress of, for example, light irradiation, a nutrient starvation state or presence of an oxide causes some photosynthetic microalgae to accumulate xanthophyll etc. in cells and turn into dormant spores.

Going into the dormant state is referred to as Encystment. In the present invention, the encystment includes both a state of going into a dormant state to start accumulating xanthophyll and a state of being fully encysted to turn into dormant spores.

### (Xanthophyll content of encysted photosynthetic microalgae containing xanthophyll)

As encysted photosynthetic microalgae containing xanthophyll, which are used in the present invention, it is preferable to use photosynthetic microalgae which accumulate xanthophyll to some degree so that daughter cells are formed while the photosynthetic microalgae contain xanthophyll, and just after growth of cells by release of the daughter cells, xanthophyll can be generated by stress of light irradiation.

Specifically, the encysted photosynthetic microalgae containing xanthophyll used in the step (A), are preferably encysted photosynthetic microalgae containing xanthophyll in an amount of 3 to 9% by mass in terms of a dry mass. That is, it is preferable that the photosynthetic microalgae to be irradiated with light in the present invention contain xanthophyll in an amount of 3 to 9% by mass in terms of a dry mass just before light irradiation, i.e. at the start of light irradiation (a), and the amount of xanthophyll varies during light irradiation. Since it is generally difficult to obtain encysted photosynthetic microalgae containing a large amount of xanthophyll, it is more preferable that the encysted photosynthetic microalgae containing xanthophyll contain xanthophyll in an amount of 3 to 7% by mass in terms of a dry mass.

Examples of the method for carrying out the production method of the present invention using encysted photosynthetic microalgae containing xanthophyll in a large amount, e.g. an amount of more than 7% by mass and 9% by mass or less in terms of a dry mass may include a method in which using encysted photosynthetic microalgae containing xanthophyll in an amount of 3 to 7% by mass in terms of a dry mass, the production method of the present invention is carried out to obtain encysted photosynthetic microalgae containing xanthophyll in an amount of more than 7% by mass and 9% by mass or less in terms of a dry mass, and using the obtained encysted photosynthetic microalgae containing xanthophyll in an amount of more than 7% by mass and 9% by mass or less in terms of a dry mass, the production method of the present invention is carried out again.

The xanthophyll content of the photosynthetic microalgae can be determined from the mass of a predetermined amount of photosynthetic microalgae dried, and the content of xanthophyll contained in a predetermined amount of photosynthetic microalgae.

### [Step (A)]

The method for culturing photosynthetic microalgae according to the present invention comprises step (A) of increasing the number of cells in which light irradiation (a) of the encysted photosynthetic microalgae containing xanthophyll as described above is performed.

Step (A) is not particularly limited, and can be carried out in the same manner as in, for example, a previously known culture method that is carried out for increasing the number of cells of photosynthetic microalgae except that light irradiation (a) as described later is performed.

Step (A) is carried out by, for example, a method in which encysted photosynthetic microalgae containing xanthophyll are inoculated in a medium, and light irradiation (a) is performed.

Step (A) is carried out preferably for 3 to 7 days, more preferably for 4 to 6 days. In step (A), light irradiation (a) is normally constantly performed, but light irradiation (a) may be temporarily stopped, for example, when confirming progress of the step. However, when light irradiation is temporarily stopped, the total time during which light irradiation is not performed in step (A) is preferably 5% or less of the time of step (A).

The amount of encysted photosynthetic microalgae containing xanthophyll, which is used in step (A), is not particularly limited, but is normally 0.05 to 5 g, preferably 0.3 to 2 g, per 1L of a medium as described later. The amount of encysted photosynthetic microalgae is preferably in the above-described range because xanthophyll can be more efficiently obtained.

The number of cells of photosynthetic microalgae is increased in step (A). The present inventors supposed that the reason why the number of cells of photosynthetic microalgae is increased is as follows. Encysted photosynthetic microalgae containing xanthophyll are turned into cyst cells containing 2 to 32 daughter cells by performing photosynthesis while utilizing nutrients in the medium in step (A). Daughter cells containing xanthophyll are released from the cyst cells. In this way, it is considered that the number of cells of photosynthetic microalgae may be increased in step (A).

### [Step (B)]

The method for culturing photosynthetic microalgae according to the present invention comprises step (B) of increasing the xanthophyll content in photosynthetic microalgae light irradiation (b) of the photosynthetic microalgae subjected to the step (A) of increasing the number of cells is performed. In step (B), the xanthophyll content in individual photosynthetic microalgae is increased by performing light irradiation (b).

Step (B) is not particularly limited, and can be carried out in the same manner as in, for example, a previously known culture method which is carried out at the time of accelerating encystment for increasing the xanthophyll content of photosynthetic microalgae except that light irradiation (b) described later is performed.

Step (B) is carried out by, for example, a method in which after step (A) is carried out, light irradiation (b) is performed without taking out photosynthetic microalgae, or a method in which after step (A) is carried out, photosynthetic microalgae are taken out, and then inoculated in a new medium, and light irradiation (b) is performed.

Step (B) is carried out preferably for 4 to 10 days, more preferably for 6 to 8 days. In step (B), light irradiation (b) is normally constantly performed, but light irradiation (b) may be temporarily stopped, for example, when confirming progress of the step. However, when light irradiation is temporarily stopped, the total time during which light irradiation is not performed in step (B) is preferably 5% or less of the time of step (B).

In addition, in the culture method of the present invention, step (A) and step (B) are carried out preferably for 7 to 17 days in total, more preferably for 10 to 14 days in total.

In step (B), the xanthophyll content in photosynthetic microalgae is increased. The present inventors supposed that the reason why the xanthophyll content is increased is as follows. It is considered that in photosynthetic microalgae having an increased number of cells through step (A), encystment is advanced by stress of nutrient starvation and light irradiation in step (B), so that additional xanthophyll is generated and accumulated in the cells of the photosynthetic microalgae, and therefore the xanthophyll content is increased.

In the culture method of the present invention, xanthophyll can be efficiently obtained because in this way, the number of cells of photosynthetic microalgae is increased, and the amount of xanthophyll in the cells is increased subsequently to the increase in the number of cells.

In the culture method of the present invention, the xanthophyll content of photosynthetic microalgae is kept at preferably 2% by mass or more, more preferably 2.5% by mass or more, still more preferably 2.8% by mass or more, in terms of a dry mass, in step (A) and step (B). That is, it is preferable that in step (A) and step (B), constantly the xanthophyll content of photosynthetic microalgae is kept at preferably 2% by mass or more, more preferably 2.5% by mass or more, still more preferably 2.8% by mass or more, in terms of a dry mass, so that daughter cells are released while containing xanthophyll, and are not killed by damage of light irradiation, and xanthophyll can be generated by stress of light irradiation. In step (A) and step (B), the upper value of the xanthophyll content of photosynthetic microalgae is not particularly limited, but is normally 15% by mass or less in terms of a dry mass.

In the culture method of the present invention, the number of cells is increased in step (A) as described above, and the xanthophyll content of photosynthetic microalgae in terms of a dry mass decreases when the number of cells is increased. Even in step (A) of increasing the number of cells, the xanthophyll content of photosynthetic microalgae in terms of a dry mass is preferably in the above-described range because xanthophyll can be more efficiently obtained.

### (Light irradiation (a) and light irradiation (b))

In step (A), light irradiation (a) is performed. The light irradiation (a) is light irradiation using as a light source an LED including blue light having a wavelength of 400 to 490 nm. The light irradiation (a) is preferably at least one light irradiation selected from light irradiation (I) using a white LED as a light source, light irradiation (II) using a white LED and a blue LED as a light source, light irradiation (III) using a white LED and a red LED as a light source, light irradiation (IV) using a blue LED and a red LED as a light source, light irradiation (V) using a blue LED and a red LED alternately as a light source, and light irradiation (VI) using a blue LED as a light source.

In step (B), light irradiation (b) is performed. The light irradiation (b) is light irradiation using as a light source an LED including blue light having a wavelength of 400 to 490 nm and an LED including red light having a wavelength of 620 to 690 nm. The LED including blue light and the LED including red light may be different LEDs, or the same LED. That is, for example, as the LED including blue light and the LED including red light, a white LED including blue light and red light can be used. The light irradiation (b) is at least one light irradiation selected from light irradiation (I) using a white LED as a light source, light irradiation (II) using a white LED and a blue LED as a light source, light irradiation (III) using a white LED and a red LED as a light source, light irradiation (IV) using a blue LED and a red LED as a light source, and light irradiation (V) using a blue LED and a red LED alternately as a light source.

A white LED including at least blue light is used when light irradiation (I) is performed in light irradiation (a), a white LED including at least blue light and red light is used when light irradiation (I) is performed in light irradiation (b), and a white LED including at least red light is used when light irradiation (II) is performed in light irradiation (b).

In the present invention, light irradiation (a) and light irradiation (b) are light irradiations using different light sources. The term "light irradiations using different light sources" means satisfying at least one of the following requirements: "light irradiation is performed using light sources (LEDs) with different emission wavelengths as at least some light sources in light irradiation (a) and light irradiation (b)" and "light irradiation is performed in which the intensity ratio of light sources in light irradiation (a) and the intensity ratio of light sources in light irradiation (b) are different when light irradiation simultaneously using a plurality of light sources with different emission wavelengths is performed in light irradiation (a), light irradiation simultaneously using a plurality of light sources with different emission wavelengths is performed in light irradiation (b), and the combinations of light sources (emission wavelengths) used in light irradiation (a) and light irradiation (b) are the same. The term "light irradiations using different light sources" does not mean that the light amounts of light sources, the numbers of light sources (LEDs) or the like are different.

More specifically, in use of different light sources, at least some of light sources may be different when a plurality of light sources are used as light sources in at least one light irradiation. For example, when a white LED is used in light irradiation (a), light irradiation (a) and light irradiation (b) are light irradiations using different light sources when in light irradiation (b), a white LED and a blue LED are used, or a white LED and a red LED are used. As another example, when a white LED and a blue LED are used in light irradiation (a), light irradiation (a) and light irradiation (b) are light irradiations using different light sources when in light irradiation (b), a white LED is used, a white LED and a red LED are used, or a blue LED and a red LED are used. When light irradiation (a) is alternating irradiation with blue light and red light using a blue LED and a red LED, light irradiation (a) is a combination of irradiation with only blue light and irradiation with only red light, and therefore light irradiation (a) and light irradiation (b) are considered as light irradiations using different light sources when light irradiation (b) is simultaneous irradiation with blue light and red light.

In addition, another example of using different light sources is a case where when light irradiation using a blue LED and a red LED is performed in light irradiation (a) and light irradiation (b), the emission intensity of the blue LED is higher than the emission intensity of the red LED in light irradiation (a), and the emission intensity of the blue LED is lower than the emission intensity of the red LED in light irradiation (b).

The light irradiation that is performed in light irradiation (a) is preferably light irradiation (I), light irradiation (II), light irradiation (IV), light irradiation (V) or light irradiation (VI). In light irradiation (a), irradiation with blue light is effective, and it is preferable to use a blue LED and a white LED. The light irradiation (a) is preferably light irradiation (I), light irradiation (II), light irradiation (IV) or light irradiation (VI) from the viewpoint of ease of control.

The light irradiation that is performed in light irradiation (b) is preferably light irradiation (III), light irradiation (IV) or light irradiation (V). In light irradiation (b), irradiation with blue light and red light is effective, and it is preferable to use a red LED together with a blue LED and a white LED. The light irradiation (b) is preferably light irradiation (III) or light irradiation (IV) from the viewpoint of ease of control.

The blue LED is an LED (light emitting diode) with a peak wavelength of 400 to 490 nm, preferably an LED with a peak wavelength of 430 to 470 nm. As the blue LED, for example, an LED (GA2RT450G) manufactured by Showa Denko K.K. can be used.

The red LED is an LED (light emitting diode) with a peak wavelength of 620 to 690 nm, preferably an LED with a peak wavelength of 645 to 675 nm. As the red LED, for example, an LED (HRP-350F) manufactured by Showa Denko K.K. can be used.

Examples of the white LED may include white LEDs having blue LED chips combined with a phosphor in which excitation light is blue light, and the emission wavelength is in the yellow light region; white LEDs having blue LED chips combined with a phosphor in which excitation light is blue light, and the emission wavelength is in the yellow light region, and a phosphor in which the emission wavelength is in a region of light other than yellow light (e.g. red light, green light or blue-green light); and white LEDs having blue, red and green LED chips. As the white LED, for example, an LED (NESW146A) manufactured by Nichia Corporation or an LED (LTN40YD) manufactured by Beamtec Co., Ltd. can be used.

The light amount (intensity) in each of light irradiation (a) and light irradiation (b) is not particularly limited, but for example, the photosynthetic photon flux density (PPFD) is preferably 750 µmol/(m²·s) or more, more preferably 1,000 µmol/(m²·s) or more, especially preferably 1,200 µmol/(m²·s) or more. The upper value of the photosynthetic photon flux density is not particularly limited, but is normally 30,000 µmol/(m²·s) or less, preferably 20,000 µmol/(m²·s) or less from the viewpoint of easy of acquiring equipment and energy efficiency. When two kinds of LEDs with different emission wavelengths are simultaneously used in light irradiation, the above-described light amount is the total light amount of the LEDs used.

In light irradiation (a), light irradiation using as a light source an LED including blue light having a wavelength of 400 to 490 nm is performed. The light amount (PPFD) of blue light having a wavelength of 400 to 490 nm in light irradiation (a) is preferably 5% or more, more preferably 10% or more, still more preferably 15% or more, where the total light amount (PPFD) is 100%. The upper value of the light amount of the blue light is not particularly limited, and may be 100%. When a blue LED is used as a light source, the light amount (PPFD) of blue light having a wavelength of 400 to 490 nm is normally 80 to 100%. The light amount of the blue light is preferably in the above-described range because the number of cells of photosynthetic microalgae is suitably increased.

In light irradiation (b), light irradiation using as a light source an LED including blue light having a wavelength of 400 to 490 nm and an LED including red light having a wavelength of 620 to 690 nm is performed. The light amount (PPFD) of blue light having a wavelength of 400 to 490 nm in light irradiation (b) is preferably 5% or more, more preferably 10% or more, still more preferably 15% or more, where the total light amount (PPFD) is 100%. In addition, the light amount (PPFD) of red light having a wavelength of 620 to 690 nm in light irradiation (b) is preferably 5% or more, more preferably 10% or more, still more preferably 15% or more, where the total light amount (PPFD) is 100%. The upper value of the light amount of each of the blue light and the red light is not particularly limited, and when a blue LED and a red LED are used as a light source, the sum of the light amount of blue light having a wavelength of 400 to 490 nm and the light amount of red light having a wavelength of 620 to 690 nm may be 100%. The light amount of each of the blue light and the red light is preferably in the above-described range because the xanthophyll content of photosynthetic microalgae is suitably increased.

In a change from step (A) to step (B), i.e. a change from light irradiation (a) to light irradiation (b), it is preferable that a change occurs in at least one of blue light and red light, i.e. the light amount of blue light is decreased, or the light amount of red light is increased, and it is more preferable that a change occurs in both of blue light and red light. Occurrence of the change is preferable because the number of cells of photosynthetic microalgae is increased in light irradiation (a), and the xanthophyll content is suitably increased in light irradiation (b).

When the light amount of blue light is decreased in a change from light irradiation (a) to light irradiation (b), the ratio of the light amount of blue light to the total light amount of all light beams applied in light irradiation (b) is decreased preferably by 3% or more, more preferably by 30% or more, especially preferably by 40% or more with respect to the ratio of the light amount of blue light to the total light amount of all light beams applied in light irradiation (a).

When the light amount of red light is increased in a change from light irradiation (a) to light irradiation (b), the ratio of the light amount of red light to the total light amount of all light beams applied in light irradiation (b) is increased preferably by 10% or more, more preferably by 15% or more, especially preferably by 25% or more with respect to the ratio of the light amount of red light to the total light amount of all light beams applied in light irradiation (a).

In addition, where ΔB is a difference between the light amount of blue light in the light irradiation (b) and the light amount of blue light in the light irradiation (a) (light amount of blue light in light irradiation (b) - light amount of blue light in light irradiation (a)), and ΔR is a difference between the light amount of red light in the light irradiation (b) and the light amount of red light in the light irradiation (a) (light amount of red light in light irradiation (b) - light amount of red light in light irradiation (a)), it is preferable that the relationship of ΔR - ΔB > 0 is satisfied from the viewpoint of the xanthophyll content.

When light irradiation (V) is performed, the light amount of blue light is 0% at the time of performing light irradiation using a red LED, and the light amount of red light is 0% at the time of performing light irradiation using a blue LED. When light irradiation (V) is performed, each of the time of light irradiation using a blue LED and the time of light irradiation using a red LED at the time of performing light irradiation (V) should be in a range as described later.

The photosynthetic photon flux density is preferably in the above-described range because the light amount is sufficient, so that photosynthetic microalgae can be grown and xanthophyll can be generated efficiently. When light irradiation is performed at a high photosynthetic photon flux density on photosynthetic microalgae which do not sufficiently contain xanthophyll, cells may be damaged and killed before the number of cells is increased, but in the culture method of the present invention, encysted photosynthetic microalgae containing xanthophyll are used as described above, and therefore the number of cells is increased even when light irradiation is performed at a high photosynthetic photon flux density. Thus, the culture method of the present invention is preferable.

The light amounts in light irradiation (a) and light irradiation (b) may be the same, or different. In step (A), the light amount in light irradiation (a) may be constant from the viewpoint of ease of control, or may be varied to be controlled to an optimum light amount according to a cell density. In step (B), the light amount in light irradiation (b) may be constant from the viewpoint of ease of control, or may be varied to be controlled to an optimum light amount according to a cell density.

In light irradiations (light irradiations (II), (III) and (IV)), the ratio of light amounts (intensities) in simultaneous use of two kinds of LEDs with different emission wavelengths is not particularly limited. The intensity ratio (ratio of photosynthetic photon flux density) of an X-color LED and a Y-color LED is normally 1 : 20 to 20 : 1, preferably 1 : 15 to 15 to 1, more preferably 1 : 10 to 10 : 1, where the X-color LED is an LED of arbitrary color, which is used for light irradiation, and the Y-color LED is an LED with an emission wavelength different from that of the X-color LED, which is used for light irradiation.

Light irradiation (V) is light irradiation using a blue LED and a red LED alternately as described above. That is, in light irradiation (V), light irradiation using a blue LED and light irradiation using a red LED are performed alternately. In light irradiation (V), light irradiation using each LED is separately and independently performed for a fixed period of time.

In light irradiation (V), light irradiation using a blue LED and light irradiation using a red LED are each performed at least once. Where I_{B} is light irradiation using a blue LED, and I_{R} is light irradiation using a red LED, light irradiation (V) is, for example, light irradiation in which I_{B} and I_{R} are performed in this order, or light irradiation in which a step including light irradiation in which I_{B} and I_{R} are performed in this order is carried out at least once.

In light irradiation (V), the ratio of the time for performing I_{B} and the time for performing I_{R} is not particularly limited. When light irradiation (V) is performed as light irradiation (a), the ratio of the time for performing I_{B} and the time for performing I_{R} (I_{B} : I_{R}) is normally 1 : 1 to 250 : 1. In addition, when light irradiation (V) is performed as light irradiation (b), the ratio of the time for performing I_{B} and the time for performing I_{R} (I_{B} : I_{R}) is normally 1 : 1 to 1 : 250.

The time for performing I_{B} means the total time of light irradiation using a blue LED, which is performed in step (A) or step (B), when light irradiation using a blue LED is performed multiple times, and the time for performing I_{R} means the total time of light irradiation using a red LED, which is performed in step (A) or step (B), when light irradiation using a red LED is performed multiple times.

Light irradiation (a) is at least one light irradiation selected from light irradiations (I) to (VI), and light irradiation (a) may be one light irradiation selected from light irradiations (I) to (VI), or may include two or more light irradiations selected from light irradiations (I) to (VI). Light irradiation (a) is preferably one light irradiation selected from light irradiations (I) to (VI) from the viewpoint of control.

The phrase "light irradiation (a) includes two or more light irradiations selected from light irradiations (I) to (VI)" means, for example, an aspect in which two or more light irradiations selected from light irradiations (I) to (VI) are performed simultaneously or sequentially.

Light irradiation (b) is at least one light irradiation selected from light irradiations (I) to (V), and light irradiation (b) may be one light irradiation selected from light irradiations (I) to (V), or may include two or more light irradiations selected from light irradiations (I) to (V). Light irradiation (b) is preferably one light irradiation selected from light irradiations (I) to (V) from the viewpoint of control.

The phrase "light irradiation (b) includes two or more light irradiations selected from light irradiations (I) to (V)" means, for example, an aspect in which two or more light irradiations selected from light irradiations (I) to (V) are performed simultaneously or sequentially.

As described above, in the present invention, light irradiation (a) and light irradiation (b) are light irradiations using different light sources, and when the above-described two or more light irradiations are performed in at least one of light irradiation (a) and light irradiation (b), at least some of light sources used in light irradiation (a) and light irradiation (b) may be different. For example, when light irradiation (I) and light irradiation (II) are performed in light irradiation (a), light irradiation (a) and light irradiation (b) are light irradiations using different light sources when in light irradiation (b), only light irradiation (I) is performed, only light irradiation (II) is performed, light irradiation (I) and light irradiation (III) or (IV) are performed, light irradiation (II) and light irradiation (III) or (IV) are performed, or the like.

In addition, light irradiation (a) and light irradiation (b) are considered as light irradiations using different light sources when light irradiation (IV) is performed in both light irradiation (a) and light irradiation (b) as described above, and the emission intensities of a blue LED and a red LED in light irradiation (a) are different from the emission intensities of a blue LED and a red LED in light irradiation (b).

Hereinafter, conditions other than those for light irradiation (a) and light irradiation (b) at the time of carrying out step (A) and step (B) will be described.

### (Medium)

The medium to be used in the method for culturing photosynthetic microalgae according to the present invention is not particularly limited.

As the medium, a liquid medium containing nitrogen necessary for growth of photosynthetic microalgae, and inorganic salts of a very small amount of metals (e.g. phosphorus, potassium, magnesium and iron) is normally used.

As the medium, specifically, a medium such as a VT medium, a C medium, an MC medium, an MBM medium or an MDM medium (see "Methods in Phycological Studies", edited by Mitsuo Chihara and Kazutoshi Nishizawa, Kyoritsu Shuppan Co., Ltd. (1979)), an OHM medium, a BG-11 medium, or a modified medium thereof is used.

In step (A) of increasing the number of cells, the number of cells of photosynthetic microalgae is increased, i.e. the cells of photosynthetic microalgae are grown. The medium to be used in step (A) of increasing the number of cells is preferably a medium, to which a component serving as a nitrogen source suitable for growth is added, e.g. a medium having a nitrogen concentration of 0.03 g/L or more, preferably 0.03 to 0.5 g/L, more preferably 0.05 to 0.5 g/L.

The nitrogen concentration is preferably in the above-described range because the number of cells can be efficiently increased, and the content of xanthophyll in photosynthetic microalgae can be sufficiently increased even when the medium used in step (A) is used as such in step (B).

A medium is normally used in step (B), and as the medium to be used in step (B), the medium used in step (A) may be used as such, or a medium different from the medium in step (A) may be used. From the viewpoint of increasing the content of xanthophyll in photosynthetic microalgae, the medium to be used in step (B) is preferably a medium containing little component serving as a nitrogen source, e.g. a medium having a nitrogen concentration of less than 0.02 g/L, preferably less than 0.01 g/L.

When the medium used in step (A) is used as such, the concentration of nitrogen contained in the medium is normally less than 0.02 g/L at the time when increase of the number of cells is stopped, or substantially stopped, in step (A). In addition, when different media are used in step (A) and in step (B), a medium having the above-described nitrogen concentration may be employed in step (B).

The nitrogen concentration is preferably in the above-described range because the content of xanthophyll in photosynthetic microalgae can be sufficiently increased in step (B).

### (Culture conditions)

The culture conditions in step (A) and step (B) are not particularly limited, and a temperature and a pH which are generally employed in culture of photosynthetic microalgae are employed.

Photosynthetic microalgae are cultured at, for example, 15 to 35°C, preferably 20 to 30°C, more preferably 22 to 28°C. The pH during culture is kept at preferably 6.0 to 10.0, more preferably 7.0 to 9.0.

Preferably, carbon dioxide is supplied in step (A) and step (B). Carbon dioxide is supplied by blowing a gas containing carbon dioxide at a concentration of 1 to 5 V/V% in such a manner that the flow rate is, for example, 0.2 to 2 vvm. As the gas containing carbon dioxide, a gas of mixed carbon dioxide and air, or a gas of mixed carbon dioxide and nitrogen gas can be used.

When a flat culture vessel such as a flat culture bottle is used, the culture liquid is stirred by the supply of carbon dioxide, so that light irradiation is uniformly performed on microalgae. Stirring of the culture liquid may be separately performed using a stirrer.

### (Culture apparatus)

The culture apparatus to be used in step (A) and step (B) is not particularly limited, and may be an apparatus capable of performing light irradiation of photosynthetic microalgae, normally a culture liquid containing photosynthetic microalgae, but the culture apparatus normally has a line through which a gas containing carbon dioxide can be supplied.

As the culture apparatus, for example, a flat culture bottle is used in the case of a small-scale culture apparatus, and a flat culture vessel composed of a transparent plate made of glass, plastic or the like, a tank-type culture vessel provided with an illuminator and a stirrer, a tubular culture vessel, an airdome-type culture vessel, a hollow cylindrical culture vessel or the like is used in the case of a large-scale culture apparatus. In addition, an airtight container is preferably used.

### (Culture method)

In the method for culturing photosynthetic microalgae according to the present invention, a medium, culture conditions, a culture apparatus and the like as described above are appropriately selected and combined, and step (A) and step (B) are carried out. Methods for carrying out step (A) and step (B) are classified broadly into two methods. The first method is a method in which step (A) and step (B) are carried out using the same medium, i.e. a one-stage culture method. The second method is a method in which after step (A) is carried out, photosynthetic microalgae are separated from a medium, and step (B) is carried out using the separated photosynthetic microalgae and a new medium, i.e. a two-stage culture method. The one-stage culture method is preferable in that since the same medium is used in step (A) and in step (B), operation is facilitated, and since step (A) and step (B) are successively carried out, contamination of unwanted bacteria hardly occurs. The two-stage culture method is preferable in that an optimum medium can be selected in each of step (A) and step (B). The one-stage culture method is not suitable for continuous culture, and is normally carried out as batch-type culture.

### (Productivity and culture liquid)

The method for culturing photosynthetic microalgae according to the present invention includes step (A) and step (B), so that xanthophyll can be obtained more efficiently than before.

Specifically, the xanthophyll productivity (mg/(L·day)) obtained by dividing the amount of xanthophyll (mg) obtained through the step (B) per 1 L of a culture liquid of photosynthetic microalgae by the total period (days) during which the steps (A) and (B) are carried out is preferably 20 mg/(L·day) or more, more preferably 30 mg/(L·day) or more, especially preferably 40 mg/(L·day) or more. The productivity is preferably as high as possible, and the upper value of the productivity is not particularly limited, but in the culture method of the present invention, the xanthophyll productivity is normally 100 mg/(L·day) or less.

In the method for culturing photosynthetic microalgae according to the present invention, a liquid medium is normally used, and therefore a culture liquid of photosynthetic microalgae is obtained. The xanthophyll content of the culture liquid of photosynthetic microalgae, which is obtained in the culture method of the present invention, is preferably 300 mg/L or more, more preferably 400 mg/L or more, especially preferably 500 mg/L or more per 1L of the culture liquid. The xanthophyll content is preferably as high as possible, and the upper value of the xanthophyll content is not particularly limited, but the xanthophyll content of the resulting culture liquid of photosynthetic microalgae is normally 1000 mg/L or less.

The photosynthetic microalgae obtained by the method for culturing photosynthetic microalgae according to the present invention contain xanthophyll in an amount of preferably 4 to 15% by mass, more preferably 5 to 12% by mass or more in terms of a dry mass.

### (Recovery of xanthophyll)

In the method for culturing photosynthetic microalgae according to the present invention, xanthophyll is accumulated in photosynthetic microalgae. Thus, the method for recovering xanthophyll after recovery of photosynthetic microalgae is not particularly limited, and xanthophyll is recovered from photosynthetic microalgae by a method such as a previously known method. Examples of the method for recovering xanthophyll from photosynthetic microalgae include a method in which photosynthetic microalgae are mechanically broken, and then extracted with an organic solvent or supercritical carbon dioxide.

### Examples

The present invention will now be described in further detail by showing examples, but the present invention is not limited to these examples.

### (Photosynthetic microalgae)

As photosynthetic microalgae, a *Haematococcus lacustris* NIES-144 strain was used.

### (Measurement of astaxanthin concentration in culture liquid)

A predetermined amount of a culture liquid was taken in BioMasher IV (manufactured by Nippi, Inc.), acetone was added, cells were crushed by FastPrep-24 (manufactured by Funakoshi Co., Ltd.), and astaxanthin was extracted.

The extract was centrifuged, the supernatant was then appropriately diluted with acetone, an absorbance at 474 nm was then measured, and an astaxanthin concentration (mg/L) in the culture liquid was calculated from an absorbance index (A_{1%} = 2,100) of astaxanthin in acetone.

### (Measurement of dry alga body mass)

A predetermined amount of the culture liquid was subjected to suction filtration using GS25 Glass Fiber Filter Paper (manufactured by Toyo Roshi Kaisha, Ltd.), the weight of which had been made constant in a constant-temperature drier in advance, and the filtrate was washed with ionexchange water, and then dried in a constant-temperature drier at 105°C for 2 hours. Thereafter, the dried product was cooled to room temperature in a desiccator, and a mass thereof was measured to determine a dry alga body mass (mg/L) in the culture liquid.

### (Calculation of astaxanthin concentration in cells)

The astaxanthin concentration (mg/L) in the culture liquid was divided by the dry alga body mass (mg/L) in the culture liquid to calculate an astaxanthin concentration (% by mass) in cells.

### (Measurement of total nitrogen concentration (mg/L) in culture liquid)

A supernatant was prepared by removing cells as precipitates from a predetermined amount of the culture liquid by centrifugation, and a total nitrogen concentration in the supernatant was measured using Total Nitrogen Measurement Reagent Kit 143C191 (manufactured by DKK-TOA CORPORATION) and Portable Simple Total Nitrogen/Total Phosphorus Meter TNP-10 (manufactured by DKK-TOA

### CORPORATION).

### (Measurement of the number of cells)

Using an improved Neubauer hemocytometer, the number of cells in a predetermined amount of the culture liquid was counted under a microscope to calculate the number of cells in the culture liquid (cells/mL).

### [Example 1]

400 ml of a medium as shown in Table 1 was placed in a flat culture bottle with a capacity of 1.0 L (flask thickness: about 38 mm including a glass thickness), and subjected to autoclave sterilization, and encysted *Haematococcus lacustris* NISE-144 was then inoculated at a concentration of 0.50 g/L. The astaxanthin content per dry mass of the inoculated *Haematococcus lacustris* NISE-144 was 4.8% by mass.

### [Table 1]

**Table 1**

| Components | g/L |
|---|---|
| KNO₃ | 0.7 |
| K₂HPO₄ | 0.07 |
| MgSO₄ ·7H₂O | 0.131 |
| CaCl₂·2H₂O | 0.063 |
| Citric acid (anhydrous) | 0.0105 |
| Iron (III) ammonium citrate | 0.0105 |
| EDTA·2Na | 0.00175 |
| Na₂CO₃ | 0.035 |
| H₃BO₃ | 0.005 |
| MnCl₂ ·4H₂O | 0.0032 |
| ZnSO₄ ·7H₂O | 0.0004 |
| Co(NO₃)₂ · 6H₂O | 0.000004 |
| CuSO₄·5H₂O | 0.000014 |
| (NH₄)₆Mo₇O₂₄ ·4H₂O | 0.000026 |

### <Step A>

Light irradiation (light irradiation (a)) was performed from both sides of the flat culture bottle using a blue LED (GA2RT450G manufactured by Showa Denko K.K.) (including blue light having a wavelength of 400 to 490 nm in an amount of 98% in terms of PPFD as an emission wavelength), and simultaneously, air containing 3 V/V% carbon dioxide was blown at 0.5 vvm from the bottom surface of the culture bottle to stir the culture liquid. In this state, culture was performed at 25°C.

The intensity of applied light was measured at a surface of the flat culture bottle using a light quantum meter (LI-250A manufactured by LI-COR, Inc.), and adjusted so that the photosynthetic photon flux density (PPFD) was 1,300 µmol/(m²·s) in total on both sides.

On the fifth day after the start of light irradiation, the total nitrogen concentration in the culture liquid became less than 20 mg/L. At this point, it was determined that the nitrogen source necessary for increasing the number of cells had been sufficiently consumed.

### <Step (B)>

Subsequently, PPFD was not changed, and the light source was changed from the blue LED to a white LED (LTN40YD manufactured by Beamtec Co., Ltd.) (including blue light having a wavelength of 400 to 490 nm in an amount of 19% in terms of PPFD and red light having a wavelength of 620 to 690 nm in an amount of 14% in terms of PPFD as an emission wavelength) and a red LED (HRP-350F manufactured by Showa Denko K.K.) (including red light having a wavelength of 620 to 690 nm in an amount of 96% in terms of PPFD as an emission wavelength) (with a photon flux density ratio of 5 : 1) (Example 1-1), or changed to a blue LED and a red LED (with a photon flux density ratio of 1 : 1) (Example 1-2) (light irradiation (b)). In this state, the culture was performed for 12 days after the start of culture (7 days after changing the light source).

The culture liquid was appropriately sampled, and a pH, the number of cells in the culture liquid, an astaxanthin concentration in the culture liquid, a dry alga body mass in the culture liquid and a total nitrogen concentration in the culture liquid were measured. An astaxanthin concentration in cells was calculated from the measured astaxanthin concentration in the culture liquid and the measured dry alga body mass in the culture liquid. The pH was 7.5 to 8.5 throughout the culture period.

For the number of cells in the culture liquid, the astaxanthin concentration in the culture liquid and the dry alga body mass in the culture liquid, after the end of culture, the content of water evaporated by air blow stirring was determined by calculation from the amount of the culture liquid at the beginning of starting the experiment, the amount of the culture liquid remaining in the flat culture bottle at the end of the experiment, and the amount of the culture liquid sampled in the middle of culture, and the value was corrected on the assumption that water had been evaporated at a constant rate during the culture period.

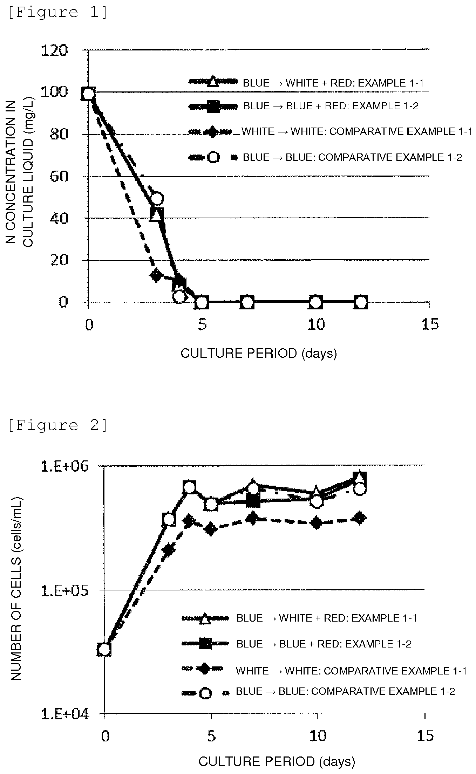

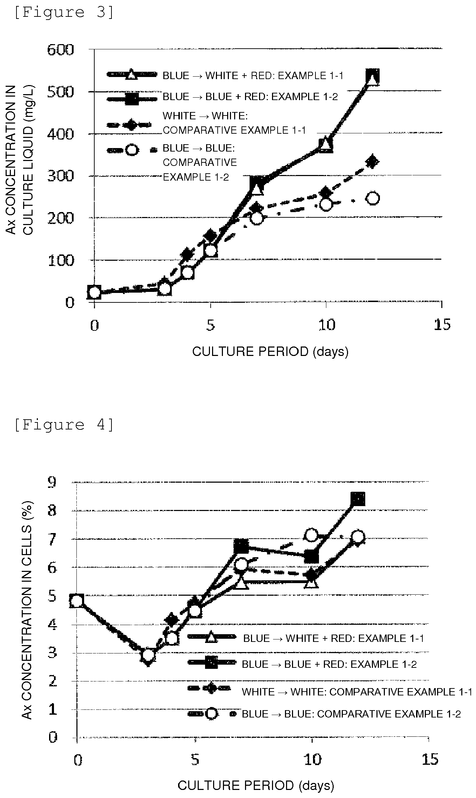

A time-dependent change of the total nitrogen concentration in the culture liquid, a time-dependent change of the number of cells in the culture liquid, a time-dependent change of the astaxanthin concentration in the culture liquid and a time-dependent change of the astaxanthin concentration in cells are shown in Figures 1, 2, 3 and 4, respectively.

Nitrogen in the culture liquid was consumed by the fifth day. The number of cells increased until the fourth day, and subsequently remained substantially constant or slightly increased, and the number of cells on the twelfth day was about 8 × 10⁵ cells/ml in both Examples 1-1 and 1-2. The astaxanthin concentration during culture was 120 mg/L at the fifth day, subsequently increased in both Examples 1-1 and 1-2, and reached 530 mg/L (astaxanthin productivity: 44 mg/(L·day)) in Example 1-1 or 540 mg/L (astaxanthin productivity: 45 mg/(L·day)) in Example 1-2 on the twelfth day. The astaxanthin concentration in cells was 4.8% by mass at the beginning of the start of culture, and decreased to the lowest concentration of 2.9% by mass on the third day, but subsequently turned to increase, and reached 7.1% by mass in Example 1-1 or 8.4% by mass in Example 1-2 on the twelfth day.

The type of the light source, the astaxanthin concentration in the culture liquid after 12 days of culture, and the productivity of astaxanthin are shown in Table 2.

### [Comparative Example 1]

Culture was performed in the same manner as in Example 1 except that light irradiation was performed from both sides of a flat culture bottle using a white LED (Comparative Example 1-1) or a blue LED (Comparative Example 1-2) so that that the total PPFD was 1,300 µmol/(m²·s), and the light source was not changed in the middle of culture.

A time-dependent change of the total nitrogen concentration in the culture liquid, a time-dependent change of the number of cells in the culture liquid, a time-dependent change of the astaxanthin concentration in the culture liquid and a time-dependent change of the astaxanthin concentration in cells are shown in Figures 1, 2, 3 and 4, respectively.

Nitrogen in the culture liquid was consumed by the fifth day. The number of cells increased until the fourth day, and subsequently remained substantially constant, and the number of cells on the twelfth day was about 7 × 10⁵ cells/ml in Comparative Example 1-2 or about 3.8 × 10⁵ cells/ml in Comparative Example 1-1. The number of cells in Comparative Example 1-1 was approximately half as large as the number of cells in Comparative Example 1-2.

The astaxanthin concentration in the culture liquid was about 120 mg/L in Comparative Example 1-2 or about 160 mg/L in Comparative Example 1-1 (white) on the fifth day. Subsequently, the astaxanthin concentration gradually increased in both Comparative Examples 1-1 and 1-2, and reached 245 mg/L (astaxanthin productivity: 20 mg/(L·day)) in Comparative Example 1-2 and 330 mg/L (astaxanthin productivity: 28 mg/(L·day)) on the twelfth day. The astaxanthin concentration in cells was 4.8% by mass at the beginning of the start of culture, and decreased to the lowest concentration of 2.9% by mass in Comparative Example 1-2 or 2.7% by mass in Comparative Example 1-1 on the third day, but subsequently turned to increase, and reached 7.1% by mass in Comparative Example 1-2 or 7.0% by mass in Comparative Example 1-1 on the twelfth day.

The type of the light source, the astaxanthin concentration in the culture liquid after 12 days of culture, and the astaxanthin productivity are shown in Table 2.

### [Example 2]

Culture was performed in the same manner as in Example 1 except that the light source for light irradiation (a), which was used at the start of culture, was changed from the blue LED to a white LED.

Example 2-1 was an example in which the light source for light irradiation (b) was changed to a white LED and a red LED (with a photon flux density ratio of 5 : 1) after elapse of 5 days after the start of light irradiation, and Example 2-2 was an example in which the light source was changed to a blue LED and a red LED (with a photon flux density ratio of 1 : 1) after elapse of 5 days after the start of light irradiation.

The type of the light source, the astaxanthin concentration in the culture liquid after 12 days of culture, and the astaxanthin productivity are shown in Table 2.

### [Example 3]

Culture was performed in the same manner as in Example 1 except that the light source for light irradiation (a), which was used at the start of culture, was changed from the blue LED to a white LED and a blue LED (with a photon flux density ratio of 5 : 1).

Example 3-1 was an example in which the light source for light irradiation (b) was changed to a white LED and a red LED (with a photon flux density ratio of 5 : 1) after elapse of 5 days after the start of light irradiation, and Example 3-2 was an example in which the light source was changed to a blue LED and a red LED (with a photon flux density ratio of 1 : 1) after elapse of 5 days after the start of light irradiation.

The type of the light source, the astaxanthin concentration in the culture liquid after 12 days of culture, and the astaxanthin productivity are shown in Table 2.

### [Example 4]

Culture was performed in the same manner as in Example 1-1 except that the light source for light irradiation (a), which was used at the start of culture, was changed from the blue LED to a blue LED and a red LED (with a photon flux density ratio of 1 : 1).

The type of the light source, the astaxanthin concentration in the culture liquid after 12 days of culture, and the astaxanthin productivity are shown in Table 2.

### [Example 5]

Culture was performed in the same manner as in Example 1 except that the light source for light irradiation (b), which was used after elapse of 5 days after the start of light irradiation, was changed to a white LED.

The type of the light source, the astaxanthin concentration in the culture liquid after 12 days of culture, and the astaxanthin productivity are shown in Table 2.

### [Example 6]

Culture was performed in the same manner as in Example 1-2 except that light irradiation (a) using a blue LED, which was performed for 5 days after the start of culture, in Example 1 was changed to light irradiation in which 21-hour light irradiation using a blue LED and 0.1-hour light irradiation using a red LED were performed alternately and continuously for 4 days after the start of culture, the light source was changed to a blue LED and a red LED after elapse of 4 days, instead of 5 days, after the start of light irradiation, and the time of light irradiation after changing the light source was changed from 7 days to 8 days (Example 6-1).

Culture was performed in the same manner as in Example 1-2 except that light irradiation (a) using a blue LED, which was performed for 5 days after the start of culture, in Example 1 was changed to light irradiation in which 92-hour light irradiation using a blue LED and 4-hour light irradiation using a red LED were performed alternately for 4 days after the start of culture, the light source was changed to a blue LED and a red LED after elapse of 4 days, instead of 5 days, after the start of light irradiation, and the time of light irradiation after changing the light source was changed from 7 days to 8 days (Example 6-2).

The type of the light source, the astaxanthin concentration in the culture liquid after 12 days of culture, and the astaxanthin productivity are shown in Table 2.

### [Table 2]

**Table 2**

| | Light irradiation (a) | Light irradiation (b) | Astaxanthin concentration in culture liquid after culture (mg/L) | Astaxanthin productivity (mg/(L-day)) |
|---|---|---|---|---|
| Example 1-1 | Blue | White + red | 530 | 44 |
| Example 1-2 | Blue | Blue + red | 540 | 45 |
| Example 2-1 | White | White + red | 470 | 39 |
| Example 2-2 | White | Blue + red | 490 | 41 |
| Example 3-1 | White + blue | White + red | 500 | 42 |
| Example 3-2 | White + blue | Blue + red | 510 | 43 |
| Example 4 | Blue + red | White + red | 490 | 41 |
| Example 5 | Blue | White | 450 | 38 |
| Example 6-1 | Alternating irradiation (blue 21 hr/red 0.1 hr) | Blue + red | 485 | 40 |
| Example 6-2 | Alternating irradiation (blue 92 hr/red 4 hr) | Blue + red | 490 | 41 |
| Comparative Example 1-1 | White | White | 330 | 28 |
| Comparative Example 1-2 | Blue | Blue | 245 | 20 |

On the basis of the ratio of blue light and red light of the light sources used in each of the examples and comparative examples, and the ratio of photon flux densities between the light sources in each of steps A and B in each of the examples and comparative examples, a ratio of blue light (%) and a ratio of red light (%) in each of light irradiation (a) and light irradiation (b) in each of the examples and comparative examples were calculated, and values of ΔB, ΔR and ΔR - ΔB were calculated. These calculated values are shown in Table 3 together with the astaxanthin concentration in the culture liquid after culture (mg/L) (expressed as "Ax concentration" in Table 3) in each of the examples.

Since there is no difference in PPFD between step A and step B in each of the examples and comparative examples, the increase and decrease in each of the ratio of blue light (%) and the ratio of red light (%) is consistent with the increase or decrease in the light amount, and the magnitude of ΔR - ΔB is consistent regardless of whether it is determined in terms of a specific light amount or in terms of a ratio.

### [Table 3]

**Table 3**

| | Light irradiation (a) | | | Light irradiation (b) | | | Light irradiation (a) | | Light irradiation (b) | | | | | |
|---|---|---|---|---|---|---|---|---|---|---|---|---|---|---|
| | | | | | | | blue light % | red light % | blue light % | red light % | ΔB | ΔR | ΔR-ΔB | Ax concentration |
| Example 1-1 | Blue | | | | White | Red | 98 | 0 | 15.8 | 27.7 | -82.2 | 27.7 | 109.8 | 530 |
| Example 1-2 | Blue | | | Blue | | Red | 98 | 0 | 49 | 48 | -49.0 | 48.0 | 97.0 | 540 |
| Example 2-1 | | White | | | White | Red | 19 | 14 | 15.8 | 27.7 | -3.2 | 13.7 | 16.8 | 470 |
| Example 2-2 | | White | | Blue | | Red | 19 | 14 | 49 | 48 | 30.0 | 34.0 | 4.0 | 490 |
| Example 3-1 | Blue | White | | | White | Red | 32.2 | 11.7 | 15.8 | 27.7 | -16.3 | 16.0 | 32.3 | 500 |
| Example 3-2 | Blue | White | | Blue | | Red | 32.2 | 11.7 | 49 | 48 | 16.8 | 36.3 | 19.5 | 510 |
| Example 4 | Blue | | Red | | White | Red | 49 | 48 | 15.8 | 27.7 | -33.2 | -20.3 | 12.8 | 490 |
| Example 5 | Blue | | | | White | | 96 | 0 | 19 | 14 | -77.0 | 14.0 | 91.0 | 450 |
| Example 6-1 | Blue red (0.4%) | | | Blue | | Red | 97.6 | 0.4 | 49 | 48 | -48.6 | 47.6 | 96.2 | 485 |
| Example 6-2 | Blue red (4.2%) | | | Blue | | Red | 93.9 | 4 | 49 | 48 | -44.9 | 44.0 | 88.9 | 490 |
| Comparative Example 1-1 | | White | | | White | | 19 | 14 | 19 | 14 | 0.0 | 0.0 | 0.0 | 330 |
| Comparative Example 1-2 | Blue | | | Blue | | | 98 | 0 | 98 | 0 | 0.0 | 0.0 | 0.0 | 245 |

## Claims

1. A method for culturing photosynthetic microalgae, the method comprising: step (A) of increasing the number of cells in which light irradiation (a) of encysted photosynthetic microalgae containing xanthophyll is performed; and step (B) of increasing the xanthophyll content in photosynthetic microalgae in which light irradiation (b) of the photosynthetic microalgae subjected to the step (A) of increasing the number of cells is performed, wherein
the light irradiation (a) is light irradiation using as a light source an LED including blue light having a wavelength of 400 to 490 nm,
the light irradiation (b) is light irradiation using as a light source an LED including blue light having a wavelength of 400 to 490 nm and an LED including red light having a wavelength of 620 to 690 nm, and
the light irradiation (a) and the light irradiation (b) are light irradiations using different light sources.

2. The method for culturing photosynthetic microalgae according to claim 1, wherein where ΔB is a difference between the light amount of blue light in the light irradiation (b) and the light amount of blue light in the light irradiation (a), and ΔR is a difference between the light amount of red light in the light irradiation (b) and the light amount of red light in the light irradiation (a), the relationship of ΔR - ΔB > 0 is satisfied.

3. The method for culturing photosynthetic microalgae according to claim 1 or 2, wherein the light irradiation (a) is at least one light irradiation selected from light irradiation (I) using a white LED as a light source, light irradiation (II) using a white LED and a blue LED as a light source, light irradiation (III) using a white LED and a red LED as a light source, light irradiation (IV) using a blue LED and a red LED as a light source, light irradiation (V) using a blue LED and a red LED alternately as a light source, and light irradiation (VI) using a blue LED as a light source, and
the light irradiation (b) is at least one light irradiation selected from light irradiation (I) using a white LED as a light source, light irradiation (II) using a white LED and a blue LED as a light source, light irradiation (III) using a white LED and a red LED as a light source, light irradiation (IV) using a blue LED and a red LED as a light source, and light irradiation (V) using a blue LED and a red LED alternately as a light source.

4. The method for culturing photosynthetic microalgae according to any one of claims 1 to 3, wherein the encysted photosynthetic microalgae containing xanthophyll used in the step (A), are encysted photosynthetic microalgae containing xanthophyll in an amount of 3 to 9% by mass in terms of a dry mass.

5. The method for culturing photosynthetic microalgae according to any one of claims 1 to 4, wherein the xanthophyll content of the photosynthetic microalgae is kept at 2% by mass or more in terms of a dry mass in the step (A) and the step (B).

6. The method for culturing photosynthetic microalgae according to any one of claims 1 to 5, wherein the photosynthetic photon flux density is 750 µmol/(m²·s) or more in the light irradiation (a) and the light irradiation (b).

7. The method for culturing photosynthetic microalgae according to any one of claims 1 to 6, wherein the step (A) is carried out for 3 to 7 days, and the step (B) is carried out for 4 to 10 days, and the step (A) and the step (B) are carried out for 7 to 17 days in total.

8. The method for culturing photosynthetic microalgae according to any one of claims 1 to 7, wherein the xanthophyll productivity (mg/(L·day)) obtained by dividing the amount of xanthophyll (mg) per 1 L of a culture liquid of photosynthetic microalgae obtained through the step (B) by the total period (days) during which the steps (A) and (B) are carried out is 20 mg/(L·day) or more.

9. The method for culturing photosynthetic microalgae according to any one of claims 1 to 8, wherein the xanthophyll is astaxanthin, and the photosynthetic microalga is a green alga of the genus *Haematococcus.*

10. A culture liquid of photosynthetic microalgae in which the content of xanthophyll obtained by the culture method according to any one of claims 1 to 9 is 300 mg/L or more.
